# EUROPÄISCHE PATENTSCHRIFT

(11) **EP 2 383 016 B1**
(45) Veröffentlichungstag und Bekanntmachung des Hinweises auf die Patenterteilung: **23.08.2017**
(21) Anmeldenummer: 11161560.5
(22) Anmeldetag: 07.04.2011
(51) Int. Cl.: A61N 1/39, A61N 1/378

(54) **Wartungssystem zum Warten einer Energiespeicheranordnung**
Maintenance system for maintaining an energy accumulator arrangement
Système d'entretien pour l'entretien d'un agencement de stockage d'énergie

(30) Priorität: 29.04.2010 US 329102 P
(43) Veröffentlichungstag der Anmeldung: 02.11.2011
(73) Patentinhaber: BIOTRONIK SE & Co. KG, 12359 Berlin (DE)
(72) Erfinder: Dörr, Thomas, 12437, Berlin (DE); Lang, Volker, West Linn, OR 97068 (US)
(74) Vertreter: Galander, Marcus

(56) Entgegenhaltungen:
- WO-A2-2006/122198
- US-A- 5 741 307
- US-A- 5 899 923

## Beschreibung

Die vorliegende Erfindung betrifft ein Wartungssystem zum Warten einer Energiespeicheranordnung eines Implantats. Die Energiespeicheranordnung des Implantats weist eine Batterie, einen Kondensator und eine Ladevorrichtung auf, wobei die Ladevorrichtung ausgebildet ist, dem Kondensator gemäß einem Ladeprogramm elektrische Ladung von der Batterie zuzuführen.

Vorrichtungen zum Warten einer solchen Energiespeicheranordnung sind grundsätzlich bekannt und werden auch als Vorrichtungen zur Batterie- und/oder Kondensatorreformierung bezeichnet. Im Gegensatz zu einem einfachen Laden des Kondensators bzw. der Batterie wird mit einer Reformierung ein gesteuertes Laden des Kondensators bzw. der Batterie bezeichnet, bei der das Laden nach einem bestimmten Ladeprogramm erfolgt. Ein Reformieren des Kondensators anstelle eines ungesteuerten Ladens ist notwendig, um eine möglichst lange Lebensdauer des Kondensators zu erzielen.

Bei einem Elektrolytkondensator, der häufig in Implantaten eingesetzt wird, ist auf der positiven Elektrode eine sehr dünne Oxidschicht als Isolator zwischen den beiden Elektroden vorgesehen. Zum Aufrechterhalten dieser isolierenden Oxidschicht ist ein geringer Leckstrom zwischen den beiden Elektroden notwendig. Entfällt dieser Leckstrom, da beispielsweise der Kondensator für längere Zeit nicht geladen worden ist, verliert die Oxidschicht im Wesentlichen ihre isolierende Wirkung. Das kann dazu führen, dass beim unkontrollierten Laden des Kondensators sehr hohe Ströme entstehen, die den Kondensator stark beanspruchen und ggf. irreversibel zerstören können.

Zur Erzielung einer langen Lebensdauer werden Kondensatoren von Implantaten daher gemäß einem Ladeprogramm geladen (reformiert), das in der Regel so gestaltet ist, dass der Kondensator mit einem geringen Ladestrom beaufschlagt wird, so dass sich die Oxidschicht reformieren kann und ihr isolierendes Verhalten wieder gewinnt. Eine solche Reformierung kann mehrere Sekunden bis Stunden andauern und erfolgt beispielsweise alle 30 Tage.

Verfahren zum gesteuerten Laden eines Kondensators sind grundsätzlich bekannt. Beispielsweise ist aus der Beschreibung des Patents US 6,096,062 ein Verfahren zum Laden eines Hochspannungskondensators in einem implantierbaren Gerät bekannt. Bei diesem Verfahren wird der Hochspannungskondensator auf eine Anfangsspannung geladen, die geringer ist als die Spitzenspannung des Kondensators. Anschließend wird ein Leckstrom erfasst. Unterschreitet der Leckstrom einen vorbestimmten Schwellenwert, wird der Hochspannungskondensator nicht weiter geladen. Überschreitet andererseits der Leckstrom den vorbestimmten Schwellenwert, so erfolgen ein weiteres Laden des Hochspannungskondensators bis zur Spitzenspannung und ein Halten der Spitzenspannung, so dass sich der Hochspannungskondensator reformieren kann. Ein Wartungssystem nach der Präambel von Anspruch 1 ist aus US5741307 bekannt. Problematisch bei bekannten Wartungssystemen ist, dass die Batterien und Kondensatoren der Implantate nach wie vor eine vergleichsweise geringe Lebensdauer aufweisen.

Es ist demnach eine der vorliegenden Erfindung zugrundeliegende Aufgabe, ein Wartungssystem zum Warten einer Energiespeicheranordnung eines Implantats vorzuschlagen, durch das die Lebensdauer der Energiespeicheranordnung erhöht werden kann.

Gemäß einem ersten Aspekt der vorliegenden Erfindung wird die oben genannte Aufgabe gelöst durch ein Wartungssystem zum Warten einer Energiespeicheranordnung eines Implantats, das die im Anspruch 1 angegebenen Merkmale aufweist.

Das erfindungsgemäße Wartungssystem umfasst:
- eine im Implantat enthaltene Überwachungsvorrichtung, die ausgebildet ist, einen Wert eines physikalischen Parameters der Energiespeicheranordnung zu erfassen und bereitzustellen;
- eine Auswerteeinheit zum Bewerten des erfassten Wertes des physikalischen Parameters, die ausgebildet ist, in Abhängigkeit des erfassten Wertes des physikalischen Parameters ein Bewertungsergebnissignal zu erzeugen;
- einen im Implantat enthaltenen Senderempfänger, der ausgebildet ist, den erfassten Wert des physikalischen Parameters an die Auswerteeinheit zu versenden, und das von der Auswerteeinheit in Abhängigkeit des erfassten Wertes des physikalischen Parameters erzeugte Bewertungsergebnissignal zu empfangen und bereitzustellen; und
- eine mit dem Implantat implantierbare Steuereinheit zum Steuern der Ladevorrichtung und der Überwachungsvorrichtung, die ausgebildet ist, das Ladeprogramm in Abhängigkeit des Bewertungsergebnissignals anzupassen.

Die vorliegende Erfindung schließt die Erkenntnis ein, dass Parameter für ein Reformieren von Batterien und Kondensatoren eines Implantats, wie beispielsweise ein Reformierintervall, eine Ladespannung, ein Ladestrom, eine Reformierdauer, im implantierten Zustand des Implantats bislang nicht oder nur sehr umständlich und eingeschränkt auf sich ändernde Anforderungen angepasst werden können. Problematisch ist dabei insbesondere, dass die Parameter zur Reformierung bei einer neuen Gerätegeneration in der Regel nur grob bestimmt sind und nicht an das tatsächliche Verhalten der Batterie bzw. des Kondensators angepasst sind. Vor diesem Hintergrund wird derzeit insbesondere die Reformierung des Kondensators sehr ineffizient betrieben. Mehr noch können in bestimmten Fällen, nämlich bei stark vom Soll-Verhalten abweichendem Verhalten des Kondensators bzw. der Batterie, diese grob bestimmten Reformierparameter zu einer Schädigung und möglicherweise zu einer irreversiblen Zerstörung der Komponenten führen. Dies erfolgt beispielsweise dann, wenn ein Kondensator, der einen überhöhten Leckstrom zeigt, im Rahmen einer regulären Reformierung auf seine Nennspannung aufgeladen wird. Anstelle des angestrebten Ausheilens des Oxiddefekts kann hier eine thermische Zerstörung des Kondensators die Folge sein. Ebenso erfolgt bei bekannten Wartungssystemen eine Reformierung des Kondensators, obgleich die Spannung der Batterie hierfür zu niedrig ist.

Treten die zu grob dimensionierten Parameter für die Reformierung des Kondensators bzw. der Batterie nach außen hin in Erscheinung, sind bei bekannten Wartungssystemen die Möglichkeiten zur Korrektur der Dimensionierung der Parameter für die Reformierung stark eingeschränkt oder überhaupt nicht gegeben. In der Regel muss der das Implantat tragende Patient einbestellt werden, mit der Folge, dass eine schnelle Korrektur der Dimensionierung der Reformierparameter nicht möglich ist.

Ferner haben die Erfinder erkannt, dass eine bei vielen bekannten Wartungssystemen prophylaktisch durchgeführte Kondensatorreformierung, also eine Reformierung, die durchgeführt wird, obwohl nicht notwendigerweise ein akutes Bedürfnis für eine Kondensatorreformierung besteht, zu einer nicht unerheblichen Reduktion der Lebensdauer eines Implantats, insbesondere eines ICDs (implantierbarer Kardioverter/Defibrillator), führen kann. Beispielsweise wird ein Kondensator des Implantats alle drei Monate auf die volle Nennspannung aufgeladen. Dabei wird in der Regel allenfalls die Ladezeit erfasst und bei Nichterreichen der Nennspannung innerhalb einer vorgegebenen Ladezeit der Ladevorgang abgebrochen. Das führt nach Erkenntnis der Erfinder dazu, dass sich anbahnende Kondensatorprobleme, wie beispielsweise ein zu hoher Leckstrom, nicht rechtzeitig erkannt werden können und zum anderen, dass der Kondensator, wie oben dargestellt, durch die prophylaktische Reformierung dauerhaft zerstört werden kann. Ein Kondensatorproblem wird bei bekannten Wartungssystemen demnach erst durch den frühzeitig auftretenden Ausfall des Implantats bemerkt.

Das erfindungsgemäße Wartungssystem hat den Vorteil, dass ein sich anbahnendes Batterie- und/oder Kondensatorproblem im Implantat, das sich durch einen bestimmten Wert des physikalischen Parameters bemerkbar macht, durch ein kontinuierliches und automatisches Anpassen des Ladeprogramms mit Hilfe der Überwachungsvorrichtung, der Auswerteeinheit und der Steuereinheit zeitnah behandelt werden kann bzw. können. Auf diese Weise erzielt das erfindungsgemäße Wartungssystem eine längere Lebensdauer des Implantats, eine verbesserte Betriebssicherheit sowie einen effizienteren Betrieb der Energiespeicheranordnung. Insbesondere werden durch das erfindungsgemäße Wartungssystem frühzeitig auftretende Geräteausfälle vermieden.

Ferner erlaubt das erfindungsgemäße Wartungssystem, das Ladeprogramm - und damit die Parameter für die Reformierung - bedarfsgerecht an die Anforderung der betreffenden Batterie bzw. des betreffenden Kondensators anzupassen. Für das Anpassen des Ladeprogramms muss der Patient nicht einbestellt werden. Das von der Steuereinheit angepasste Ladeprogramm ist derart konzipiert, dass durch das Laden des Kondensators gemäß dem angepassten Ladeprogramm, also durch die Reformierung des Kondensators, keine Schädigung von Kondensator oder Batterie verursacht wird.

Mit dem erfindungsgemäßen Wartungssystem sind eine individuelle Beobachtung von mehreren Energiespeicheranordnungen von im Verkehr befindlichen Implantaten sowie ein schnelles und kontinuierliches Anpassen der Ladeprogramme ohne Einbestellung der die Implantate tragenden Patienten möglich. Durch ein angepasstes Ladeprogramm laden die Ladevorrichtungen die Kondensatoren dann, wenn tatsächlich ein Reformierbedarf besteht. Eine prophylaktische Reformierung entfällt damit, wodurch Energie eingespart werden kann und das Risiko einer Schädigung der Energiespeicheranordnung gemindert ist.

Ferner erlaubt das Anpassen des Ladeprogramms ein effizientes, d.h. verlustarmes Laden des Kondensators.

Das Anpassen des Ladeprogramms erfolgt beispielsweise durch Änderung des Reformierintervalls zwischen Batterie- und/oder Kondensatorreformierungen oder durch die Änderung der Dauer der Reformierung, durch Änderung einer Ladespannung oder eines Ladestroms oder durch Änderung der Anzahl der zu ladenden Kondensatoren. In der Regel umfasst der Kondensator des Implantats eine Vielzahl parallel oder seriell verschalteter Einzelkondensatoren.

Die im Implantat enthaltene Überwachungsvorrichtung erfasst beispielsweise eine bestimmte Ladezeit, einen Wert einer Ladespannung, eine Ladespannung, einen Wert eines Ladestroms oder einen Wert eines Ladezustands oder eines sonstiges physikalischen Parameters der Energiespeicheranordnung und stellt den erfassten Wert des physikalischen Parameters bereit. Die Auswerteeinheit zum Bewerten des erfassten Wertes befindet sich wahlweise im Implantat oder außerhalb des Implantats und bewertet den Wert des physikalischen Parameters und stellt ein entsprechendes Bewertungsergebnissignal bereit.

Die im Implantat enthaltene Steuereinheit zum Steuern der Ladevorrichtung und der Überwachungsvorrichtung steht über einen ebenfalls im Implantat enthaltenen Sendeempfänger in Kommunikation mit der Auswerteeinheit. Die Überwachungsvorrichtung versendet den Wert des erfassten physikalischen Parameters mit Hilfe des Sendeempfängers an die Auswerteeinheit. Die Auswerteeinheit, beispielsweise ebenfalls ausgestattet mit einem Senderempfänger, versendet das Bewertungsergebnissignal mit Hilfe des Sendeempfängers an die Steuereinheit. Auf Basis des empfangenen Ergebnissignals nimmt die Steuereinheit ein Anpassen des Ladeprogramms vor.

Das Bewertungsergebnissignal selbst kann Soll-Reformierparameter enthalten und die Steuereinheit das Anpassen des Ladeprogramms derart vornehmen, dass sie die Ladevorrichtung gemäß den empfangenen Soll-Reformierparametern steuert. Die Auswerteeinheit ist also bevorzugt ausgebildet, ein Bewertungsergebnissignal in Gestalt von Soll-Reformierparametern zu erzeugen und bereitzustellen.

Dadurch, dass die Steuereinheit zum Steuern der Ladevorrichtung und der Überwachungsvorrichtung ausgebildet ist, kann ein mit dem Ladevorgang des Kondensators zeitlich abgestimmtes Erfassen des physikalischen Parameters erfolgen.

Im Folgenden werden weitere vorteilhafte Ausführungsformen des erfindungsgemäßen Wartungssystems beschrieben. Die zusätzlichen Merkmale dieser Ausführungsformen können miteinander und/oder mit den oben bereits genannten optionalen Merkmalen des Wartungssystems zur Bildung neuer Ausführungsbeispiele kombiniert werden, sofern sie nicht ausdrücklich als alternativ zueinander beschrieben sind.

Der Kondensator ist beispielsweise ein Elektrolytkondensator, die Batterie beispielsweise eine Silber-Vanadium-Oxid Batterie.

In einer besonders bevorzugten Ausführungsform des Wartungssystems ist die Auswerteeinheit ausgebildet, eine Vielzahl von Werten des physikalischen Parameters von Energiespeicheranordnungen einer Vielzahl verschiedener Implantate zu empfangen und das Bewertungsergebnissignal im Rahmen einer Gesamtbewertung der Vielzahl Werte zu erzeugen.

In dieser Ausführungsform des Wartungssystems empfängt die Auswerteeinheit demnach die Vielzahl Werte des physikalischen Parameters von einer Vielzahl verschiedener Implantate. Die verschiedenen Implantate, die von verschiedenen Personen getragen werden, umfassen bevorzugt jeweils baugleiche Energiespeicheranordnungen. Die Auswerteeinheit befindet sich in dieser Ausführungsform des Wartungssystems bevorzugt außerhalb des Implantats und übermittelt das Bewertungsergebnissignal zumindest an eines aus der Vielzahl verschiedener Implantate. Dabei ist die Auswerteeinheit über ein wie auch immer geartetes Datennetzwerk mit den Implantaten wirkverbunden.

Durch die Gesamtbewertung der Vielzahl von Werten kann ein besseres Bewertungsergebnissignal erzeugt werden. Die Gesamtbewertung schließt beispielsweise statistische Erhebungen sowie Mittelwertbildungen oder sonstige mathematische Verfahren zum Auswerten der Vielzahl Werte und zum Erzeugen des Bewertungsergebnissignals ein. Der Vorteil dieser Ausführungsform liegt insbesondere darin, dass das Bewertungsergebnissignal nicht nur in Abhängigkeit des einzigen Wertes des physikalischen Parameters erzeugt wird, sondern in Abhängigkeit der Vielzahl Werte des physikalischen Parameters von bevorzugt baugleichen Energiespeicheranordnungen. Somit können die oben genannten Vorteile des Wartungssystems nicht nur für ein einziges Implantat erzielt werden, sondern für die Vielzahl Implantate, die verschiedenen Personen implantiert sind.

Dazu ist die Auswerteeinheit bevorzugt über ein Patientengerät und den Sendeempfänger mit der Überwachungsvorrichtung und der Steuereinheit wirkverbunden. Somit kann der Sendeempfänger mit geringer Leistung betrieben werden, da dieser lediglich einen Datentransfer zwischen Patientengerät, das sich in der Regel in der Nähe des Patienten befindet, und dem Implantat sicherstellt.

Insgesamt ist damit ein flächendeckendes Anpassen des Ladeprogramms, also eine flächendeckende Änderung des Reformierschemas, ohne Einbestellung einer Vielzahl von Patienten möglich.

Die Steuereinheit des Wartungssystems ist in einer Ausführungsform ausgebildet, das Ladeprogramm in Abhängigkeit des Bewertungsergebnissignals durch Änderung einer Ladespannung und/oder eines Ladeintervalls und/oder eines Ladestroms anzupassen. Durch ein derartiges Anpassen des Ladeprogramms kann die Lebensdauer der Energiespeicheranordnung und damit die Lebensdauer des Implantats signifikant erhöht werden.

In einer weiteren besonders bevorzugten Ausführungsform des Wartungssystems ist die Auswerteeinheit ausgebildet, zum Erzeugen des Bewertungsergebnissignals auf eine Datenbank mit Herstellungsdaten zuzugreifen, welche den betreffenden Implantatstyp charakterisieren. Bei dieser Ausführungsform befindet sich die Auswerteeinheit bevorzugt außerhalb des Implantats und steht beispielsweise über einen Fernüberwachungsserver in Kommunikation mit der Datenbank. Diese Ausführungsform hat den Vorteil, dass das Bewertungsergebnissignal nicht nur in Abhängigkeit des Wertes bzw. der Werte des physikalischen Parameters erfolgt, sondern dass ferner das betreffende Implantat charakterisierende Herstellungsdaten berücksichtigt werden können und insofern ein gezielteres Anpassen des Ladeprogramms erfolgen kann.

Befindet sich die Auswerteeinheit in einem großen Abstand zum zu wartenden Implantat, so kommuniziert die Auswerteeinheit mit der Steuereinheit und der Überwachungsvorrichtung bevorzugt über ein funk- oder drahtgebundenes Datennetzwerk, wie beispielsweise das Internet.

Die Auswerteeinheit erzeugt das Bewertungsergebnissignal selbständig oder auch in Abhängigkeit manueller Eingaben.

In einer anderen Ausführungsform ist die Auswerteeinheit im Implantat selbst enthalten. In dieser Ausführungsform empfängt die Auswerteeinheit bevorzugt Daten anderer Implantate und/oder Daten aus der Herstellungsdatenbank über den Sendeempfänger und erzeugt das Bewertungsergebnissignal in Abhängigkeit des erfassten Wertes des physikalischen Parameters und der empfangenen weiteren Daten.

In einer weiteren besonders bevorzugten Ausführungsform des Wartungssystems ist die Steuereinheit ausgebildet, die Ladevorrichtung und die Überwachungsvorrichtung derart zu steuern, dass ein periodisches Laden des Kondensators nach dem folgenden Ladevorgang erfolgt:
- Initiieren des Ladens des Kondensators durch die Ladevorrichtung;
- Erfassen einer elektrischen Spannung des Kondensators mittels der Überwachungsvorrichtung;
- Unterbrechen des Ladevorgangs für eine erste definierte Zeitspanne nach einem Erreichen einer ersten definierten Kondensatorspannung des Kondensators;
- Fortsetzen des Ladens des Kondensators nach Ablauf der ersten Zeitspanne;
- Erfassen des Wertes des physikalischen Parameters in Gestalt einer zweiten Zeitspanne, welche vom Fortsetzungsbeginn bis zu einem erneuten Erreichen der ersten Ladespannung andauert, mittels der Überwachungsvorrichtung;
- Versenden der erfassten zweiten Zeitspanne an die Auswerteeinheit mittels des Sendeempfängers;
- Bewerten der zweiten Zeitspanne und Bereitstellen des Bewertungsergebnissignals durch die Auswerteeinheit;
- Empfangen des Bewertungsergebnissignals mittels des Sendeempfängers und Anpassen des Ladeprogramms in Abhängigkeit des Bewertungsergebnissignals mittels der Steuereinheit.

Diese Ausführungsform des Wartungssystems reformiert die Energiespeicheranordnung des Wartungssystems des Implantats zunächst beispielsweise alle drei Monate. Das Laden des Kondensators zeichnet sich dabei insbesondere durch eine bestimmte Unterbrechung des Ladevorgangs aus, die beim Erreichen der ersten definierten Kondensatorspannung, welche bevorzugt unterhalb der Nennspannung des Kondensators liegt. Während der Unterbrechung des Ladevorgangs für die Dauer der ersten definierten Zeitspanne führt eine Selbstentladung des Kondensators zu einem Absinken der Kondensatorspannung. Nach Ablauf der ersten definierten Zeitspanne setzt die Ladevorrichtung den Ladevorgang fort. Bei diesem Laden des Kondensators wird die erste definierte Kondensatorspannung wieder erreicht, nämlich nach Ablauf der zweiten Zeitspanne. Die Dauer dieser zweiten Zeitspanne ist abhängig von den Eigenschaften des Kondensators, der Batterie und der Ladevorrichtung. Die Überwachungsvorrichtung erfasst die Dauer der zweiten Zeitspanne als Wert des physikalischen Parameters und übermittelt den Wert an die Auswerteeinheit mit Hilfe des Senderempfängers. Bevorzugt empfängt die Auswerteeinheit eine Vielzahl Werte dieser Zeitspanne von mehreren baugleichen Energiespeicheranordnungen verschiedener Implantate. Die Auswerteeinheit bewertet mindestens einen dieser Werte, bevorzugt mehrere dieser Werte, und erzeugt in Abhängigkeit der Bewertung das Bewertungsergebnissignal. Nach der Übermittlung des Bewertungsergebnissignals passt die Steuereinheit des Wartungssystems das Ladeprogramm in Abhängigkeit des Bewertungsergebnissignals an, bevorzugt durch Ändern der ersten definierten Kondensatorspannung.

Die Bewertung der Vielzahl Werte der zweiten Zeitspanne kann durch die Auswerteeinheit beispielsweise dahingehend erfolgen, dass die Auswerteeinheit ein Bewertungsergebnissignal erzeugt, nach welchem die Steuereinheit das Ladeprogramm dahingehend ändert, dass das Reformierintervall verkürzt wird, wenn eine bestimmte Anzahl von Werten der zweiten Zeitspanne einen vorgegebenen Wert überschreitet. Beispielsweise passt die Steuereinheit das Ladeprogramm derart an, dass eine Reformierung des Kondensators alle 30 Tage mit einer verminderten ersten definierten Kondensatorspannung erfolgt. Auf diese Weise kann eine frühzeitige Zerstörung des Kondensators vermieden werden.

Außerdem passt die Steuereinheit das Ladeprogramm bevorzugt derart an, dass eine Reformierung abgebrochen wird, wenn nicht innerhalb einer vorgegebenen maximalen Zeit die erste definierte Kondensatorspannung erreicht wird. In diesem Fall wiederholt die Ladevorrichtung gemäß dem Ladeprogramm eine Reformierung mit einer geminderten Kondensatorspannung, bis eine vollständige Reformierung möglich ist.

Bevorzugt ist die Steuereinheit des Wartungssystems demnach ausgebildet, auf den Empfang des Bewertungsergebnissignals hin das Ladeprogramm durch Mindern des Wertes der ersten definierten Ladespannung anzupassen, falls das Bewertungsergebnissignal anzeigt, dass die zweite Zeitspanne eine vorgegebene Zeitdauer übersteigt.

Die Steuereinheit ist weiterhin bevorzugt ausgebildet, nach dem Mindern der ersten definierten Ladespannung und einem erneuten Überschreiten der vorgegebenen Zeitdauer den Wert der geminderten ersten definierten Ladespannung erneut zu mindern, bis die zweite Zeitdauer die vorgegebene Zeitdauer nicht mehr überschreitet.

Eine Reformierung des Kondensators nach dem oben dargestellten Ladeprogramm ist für den Kondensator besonders schonend, so dass der Kondensator und damit das Implantat eine lange Lebensdauer erzielt. Außerdem wird eine Zerstörung oder eine Beschädigung des Kondensators vermieden.

In allen Ausführungsformen des Wartungssystems ist es bevorzugt, dass die Auswerteeinheit ausgebildet ist, nicht nur einen einzigen Wert des physikalischen Parameters zu bewerten, sondern eine Vielzahl Werte des physikalischen Parameters, die bevorzugt von einer Vielzahl verschiedener Implantate baugleicher Energiespeicheranordnung stammen. Auf diese Weise kann, wie oben dargestellt, eine gezieltere Bewertung erfolgen und somit das Ladeprogramm von der Steuereinheit besser in Bezug auf die oben genannten vorteilhaften Wirkungen angepasst werden.

Bevorzugt ist die Überwachungsvorrichtung ausgebildet, einen Verlauf eines Wertes einer an der Batterie anliegenden Batteriespannung als physikalischen Parameter während eines Ladevorgangs des Kondensators zu erfassen. Die Steuereinheit ist bevorzugt ausgebildet, das Ladeprogramm bei einem Bewertungsergebnissignal, das aufgrund des erfassten Batteriespannungsverlaufs indikativ für einen Wartungsbedarf ist, anzupassen.

Durch das Erfassen des Batteriespannungsverlaufs während des Ladevorgangs des Kondensators mittels der Überwachungsvorrichtung kann die Auswerteeinheit durch geeignete Bewertung des Verlaufs einen Reformierbedarf der Energiespeicheranordnung erkennen. Der Reformierbedarf kündigt sich beispielsweise durch eine Spannungsverzögerung (engl.: Voltage Delay) an. Ein Voltage Delay wird durch die Bildung relativ hochohmiger Schichten auf den Batterieanoden hervorgerufen und stellt dadurch einen Anstieg des Batterieinnenwiderstandes dar. Dieser Effekt kann erst dann erkannt werden, wenn aus der Batterie ein relativ großer Strom entnommen werden soll und dabei die Batteriespannung überproportional abfällt. Durch eine Strombelastung werden diese hochohmigen Schichten auch wieder abgebaut und so das Voltage Delay reduziert bzw. eliminiert wird. Im Falle einer ausgeprägten Spannungsverzögerung erzeugt die Auswerteeinheit ein Bewertungsergebnissignal, nach dem die Steuereinheit das Ladeprogramm für die Ladevorrichtung derart anpasst, dass die Reformierintervalle verkürzt sind und eine stufenweise und wiederholte Batteriereformierung mit geminderten Ladespannungen erfolgt.

An dieser Stelle sei erwähnt, dass im Rahmen der Beschreibung der vorliegenden Erfindung mit Batteriespannung eine Spannung bezeichnet ist, die an Ausgangsklemmen der Batterie anliegt, mit Kondensatorspannung eine Spannung, die an Kontakten des Kondensators anliegen und mit Ladespannung eine Spannung der Ladevorrichtung, mit der die Ladevorrichtung den Kondensator lädt. Freilich können zu jedem Zeitpunkt die Beträge der Batteriespannung, der Ladespannung und der Kondensatorspannung voneinander abweichen. Gewöhnlich ist die Batteriespannung in einer ähnlichen Größenordnung wie die Ladespannung und die Kondensatorspannung deutlich höher als die Batteriespannung.

Bevorzugt ist in der oben genannten Ausführungsform die Steuereinheit ausgebildet, das Ladeprogramm derart anzupassen, dass die Ladevorrichtung den Kondensator durch eine Vielzahl aufeinanderfolgender Ladezyklen mit einer verminderten Ladespannung lädt und dabei einen der Ladezyklen abbricht, falls der Wert der Kondensatorspannung in einer vorbestimmten Zeitspanne nicht einen vorbestimmten Schwellenwert erreicht.

Diese Ausführungsform hat den Vorteil, dass bei moderater Batteriebelastung die Oxidschichten zwischen den Elektroden des Kondensators, die zum Ausbilden der Spannungsverzögerung führen, systematisch reformiert werden können.

In einer weiteren bevorzugten Ausführungsform des Wartungssystems ist die Steuereinheit ausgebildet, das Ladeprogramm auf einen Empfang des Bewertungsergebnissignals hin derart anzupassen, dass die Ladevorrichtung den Kondensator auf einen Spannungspegel deutlich unterhalb einer Kondensatornennspannung kontinuierlich lädt, sodann stufenweise lädt, bis die Kondensatornennspannung erreicht ist und schließlich die Kondensatornennspannung durch zyklisches Nachladen aufrecht erhält, falls das Bewertungsergebnissignal indikativ für einen erhöhten Leckstrom des Kondensator ist.

Auch diese Ausführungsform des Wartungssystems erzielt eine erhöhte Lebensdauer der Energiespeicheranordnung und damit eine erhöhte Lebensdauer des Implantats. Durch das stufenweise erfolgende Laden des Kondensators wird der Kondensator schonend reformiert, d.h. bereits vorhandene Fehlstellen in der Oxidschicht können bei geringerer Spannung zunächst "ausheilen", ohne dass dabei bereits eine überkritische lokale Erwärmung aufgrund der Leckströme entsteht.

Weitere Vorteile der Erfindung werden anhand der Beschreibung einiger Ausführungsbeispiele anhand der Zeichnungen erläutert. In den Zeichnungen zeigen in schematischer Darstellung:
- Fig. 1:: Komponenten einer Ausführungsform des Wartungssystems;
- Fig. 2:: ein Gesamtsystem zum Fernmanagement einer Vielzahl Energiespeicheranordnungen von verschiedenen Implantaten;
- Fig. 3:: einen ersten Verlauf einer Kondensatorspannung beim Laden des Kondensators gemäß einem Ladeprogramm;
- Fig. 4:: einen zweiten Verlauf einer Kondensatorspannung beim Laden des Kondensators gemäß einem Ladeprogramm;
- Fig. 5:: einen dritten Verlauf einer Kondensatorspannung beim Laden des Kondensators gemäß einem Ladeprogramm;
- Fig. 6:: einen vierten Verlauf einer Kondensatorspannung beim Laden des Kondensators gemäß einem Ladeprogramm;
- Fig. 7:: einen fünften Verlauf einer Kondensatorspannung beim Laden des Kondensators gemäß einem Ladeprogramm;
- Fig. 8:: einen ersten Verlauf einer Batteriespannung beim Laden eines Kondensators, welcher einen erhöhten Leckstrom aufweist;
- Fig. 9:: einen zweiten Verlauf einer Batteriespannung beim Laden eines Kondensators, bei der die Batterie eine leicht ausgeprägte Spannungsverzögerung zeigt und
- Fig. 10:: einen dritten Verlauf einer Spannung einer Batterie beim Laden eines Kondensators, bei der die Batterie ausgeprägte Spannungsverzögerung zeigt.

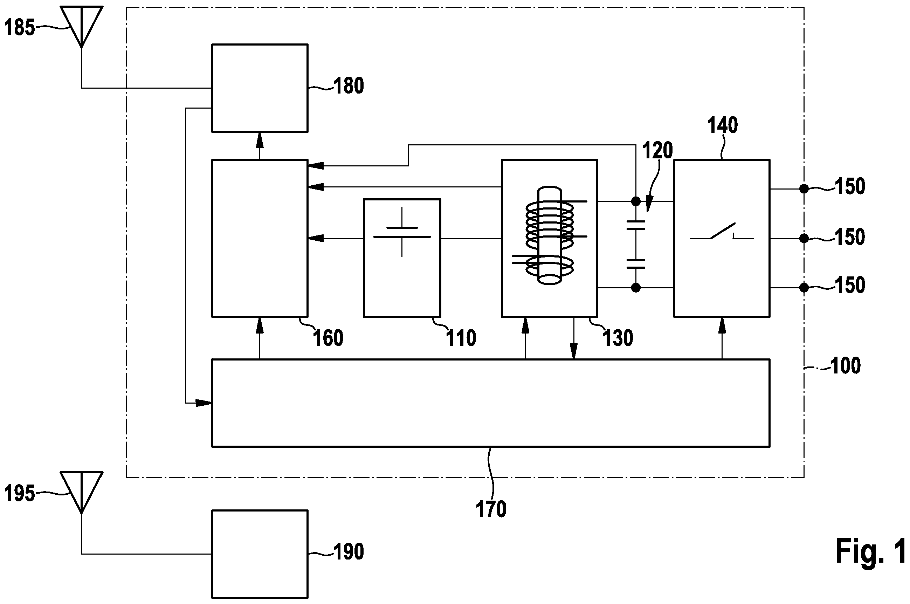

Fig. 1 zeigt in schematischer Darstellung Komponenten einer Ausführungsform des erfindungsgemäßen Wartungssystems. Das Wartungssystem nach Fig. 1 dient einem Warten einer Energiespeicheranordnung 110, 120, 130 eines Implantats 100. Die Energiespeicheranordnung weist eine Batterie 110 und einen Kondensator 120 auf. Die Batterie 110 und der Kondensator 120 sind über eine Ladevorrichtung 130 miteinander verbunden. Dem Kondensator 120 nachgeschaltet ist eine Schaltanordnung 140, über die eine Kondensatorenergie des Kondensators 120 an Schockelektroden 150 des Implantats 100 abgegeben werden kann. Das Implantat 100 ist beispielsweise ein implantierbarer Kardioverter/Defibrillator.

Die im Folgenden näher dargestellten Komponenten des Wartungssystems dienen in erster Linie dem Betreiben der Ladevorrichtung 130, so dass durch die Ladevorrichtung 130 eine lange Lebensdauer der Batterie 110 und des Kondensators 120 erzielt werden kann.

Das in Fig. 1 dargestellte Wartungssystem umfasst eine im Implantat enthaltene Überwachungsvorrichtung, die ausgebildet ist, einen Wert eines physikalischen Parameters der Energiespeicheranordnung 110, 120, 130 zu erfassen und bereitzustellen. Die Überwachungsvorrichtung ist demnach beispielsweise eine Messeinrichtung zum Erfassen einer Zeitdauer, einer Spannung der Batterie 110 (Batteriespannung), einer Spannung des Kondensators 120 (Kondensatorspannung) oder ein in der Ladevorrichtung 130 fließender Strom (Ladestrom) oder anliegende Spannung (Ladespannung).

Die Überwachungsvorrichtung 160 und die Ladevorrichtung 130 als auch die Schaltanordnung 140 werden von einer Steuereinheit 170 gesteuert. In der Steuereinheit 170 ist ein Ladeprogramm hinterlegt, nach dem die Ladevorrichtung 130 Ladung von der Batterie 110 zum Kondensator 120 führt.

Das in Fig. 1 dargestellte Wartungssystem umfasst ferner einen Sendeempfänger 180. Der Sendeempfänger 180 ist ausgebildet, den von der Überwachungsvorrichtung 160 erfassten Wert des physikalischen Parameters an eine Auswerteeinheit 190 zu versenden. Bei der in Fig. 1 dargestellten Ausfiihrungsform des Wartungssystems befindet sich die Auswerteeinheit 190 außerhalb des Implantats 100. Eine Übermittlung von Daten zwischen der Auswerteeinheit 190 und dem Implantat 100 erfolgt beispielsweise mit einem in Fig. 1 nicht näher dargestellte Patientengerät und einem funk- oder drahtgebundenen Netzwerk. Mit den Bezugszeichen 185 und 195 sind dafür notwendige Schnittstellen des Senderempfängers 180 bzw. der Auswerteeinheit 190 dargestellt. Diese arbeiten beispielsweise im GSM (Global System for Mobile Communications)- oder MICS (Medical Implant Communication Service)-Band.

Die Auswerteeinheit 190 ist ausgebildet, in Abhängigkeit des erfassten Wertes des physikalischen Parameters ein Bewertungsergebnissignal zu erzeugen und an das Implantat 100 zu senden. Der Sendeempfänger 180 empfängt das erzeugte Bewertungsergebnissignal und übermittelt dieses an die Steuereinheit 170. Die Steuereinheit 170 ist ausgebildet, in Abhängigkeit des Bewertungsergebnissignals das Ladeprogramm, nach dem die Ladevorrichtung 130 den Kondensator 120 lädt, anzupassen. Das Bewertungsergebnissignal der Auswerteeinheit 190 kann auch die Reformierparameter selbst beinhalten.

Dadurch, dass die Steuereinheit 170 auch die Überwachungsvorrichtung 160 steuert, kann ein Erfassen des Wertes des physikalischen Parameters, beispielsweise der Batteriespannung, des Ladestroms und/oder der Kondensatorspannung, zu bestimmten Zeitpunkten erfolgen.

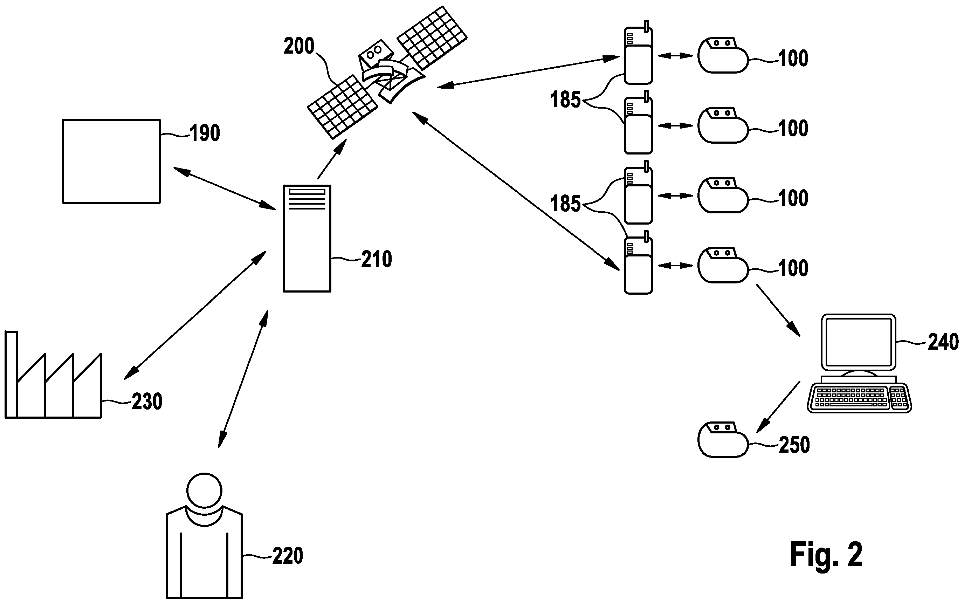

In Fig. 2 ist schematisch ein Gesamtsystem zum Fernmanagement einer Vielzahl baugleicher Energiespeicheranordnungen verschiedener Implantate 100 dargestellt. Der Aufbau eines Implantats 100 entspricht im Wesentlichen dem in Fig. 1 schematisch skizzierten Aufbau. Die Implantate 100 sind über Schnittstellen 185, die beispielsweise jeweils in einem Patientengerät untergebracht sind, an ein Datennetz 200 angeschlossen. Die Auswerteeinheit 190 des Wartungssystems ist über einen Fernüberwachungsserver 210 an dasselbe Datennetz 200 angeschlossen.

In dieser Ausführungsform ist die Auswerteeinheit 190 ausgebildet, eine Vielzahl Werte des physikalischen Parameters der Energiespeicheranordnungen der Vielzahl verschiedener Implantate 100 zu empfangen und das Bewertungsergebnissignal im Rahmen einer Gesamtbewertung dieser Vielzahl Werte zu erzeugen.

Außerdem ist das in Fig. 2 dargestellte Gesamtsystem derart konzipiert, dass eine Person 220 über den Fernüberwachungsserver 210 Einfluss nehmen kann auf die Bewertung der Vielzahl Werte des physikalischen Parameters in der Auswerteeinheit 190 und damit Einfluss nehmen kann auf das Anpassen des Ladeprogramms. Ferner kann die Auswerteeinheit 190 über den Fernüberwachungsserver 210 auf eine Datenbank 230 mit Herstellungsdaten zugreifen, die die Vielzahl Implantate 100 charakterisieren. Auf diese Weise kann ein gezielteres Anpassen des Ladeprogramms erfolgen.

Das in Fig. 2 dargestellte Gesamtsystem ist also beispielsweise derart konzipiert, dass Werte mehrerer physikalischer Parameter der Batterien- und/oder der Kondensatoren der Energiespeicheranordnungen der Implantate 100 gesammelt werden und die Auswerteeinheit 190 diese kontinuierlich und automatisch analysiert, wobei die gesammelten Daten mit Informationen aus der Datenbank 230 ergänzt werden können und außerdem eine Person 220 direkten Einfluss auf die Bewertung der Daten und damit direkten Einfluss auf die Gestaltung des Ladeprogramms nehmen kann.

Hat eines der Implantate 100 keinen Anschluss an das Datennetz 200, so ist es möglich, das angepasste Ladeprogramm eines Implantats, das an das Datennetz 200 angeschlossen ist, mit Hilfe eines Programmiergeräts 240 auszulesen und sodann an das Implantat 150 ohne Anschlussmöglichkeit an das Datennetz 200 zu übertragen.

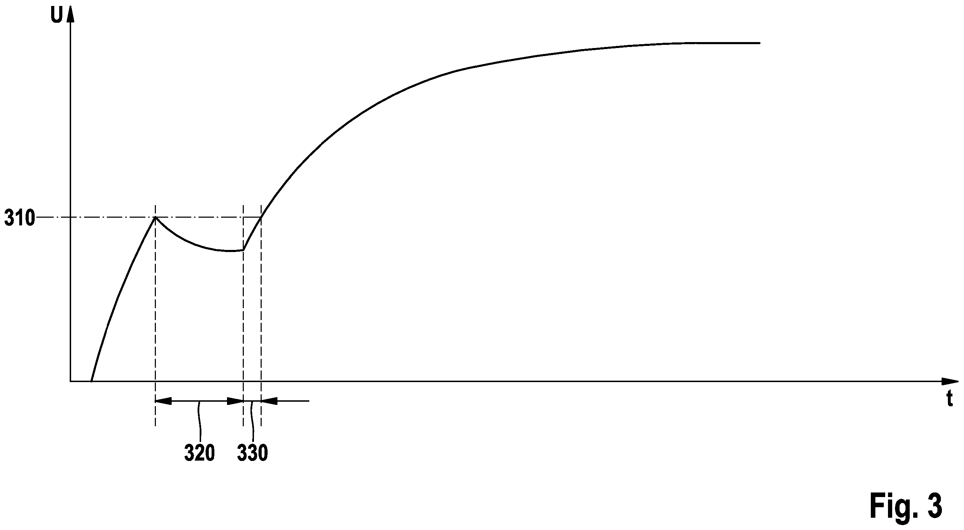

Fig. 3 zeigt in schematischer Darstellung einen ersten Verlauf des Kondensators 120 beim Laden nach einem Ladeprogramm. Demnach lädt die Ladevorrichtung 130 den Kondensator 120 bis zu einer ersten definierten Kondensatorspannung 310. Danach unterbricht die Ladevorrichtung 130 den Ladevorgang für die Dauer einer ersten definierten Zeitspanne 320. Während dieser Zeitspanne 320 sinkt die Spannung des Kondensators 120 aufgrund einer Selbstentladung. Nach Ablauf der ersten Zeitspanne 320 setzt die Ladevorrichtung 130 den Ladevorgang fort. Die Überwachungsvorrichtung 160 erfasst die Zeit, die das Laden bis zum erneuten Erreichen der ersten definierten Kondensatorspannung 310 erfordert.

Die Überwachungsvorrichtung 160 erfasst die Dauer dieser zweiten Zeitspanne 330 und versendet diese mit Hilfe des Sendeempfängers 180 an die Auswerteeinheit 190. Die zweite Zeitspanne 330 ist dabei ein Maß für die Selbstentladung des Kondensators 120.

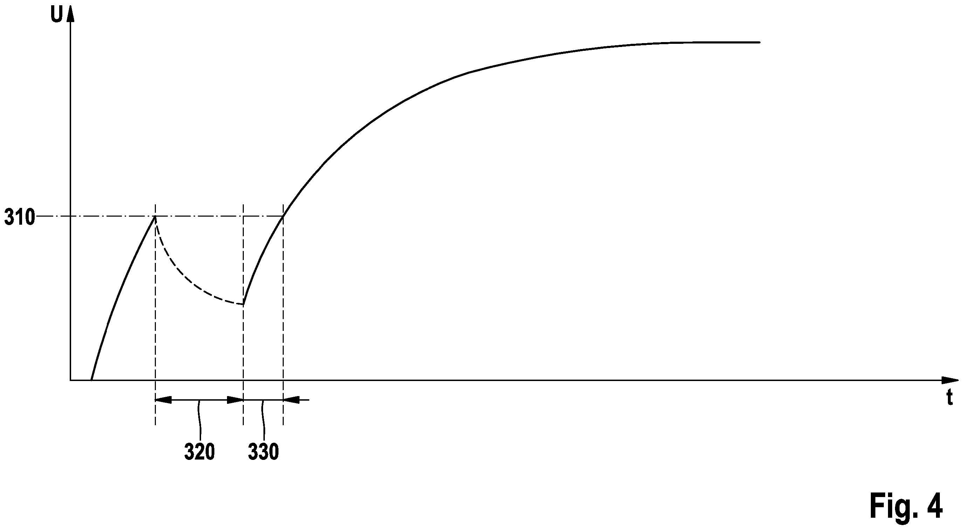

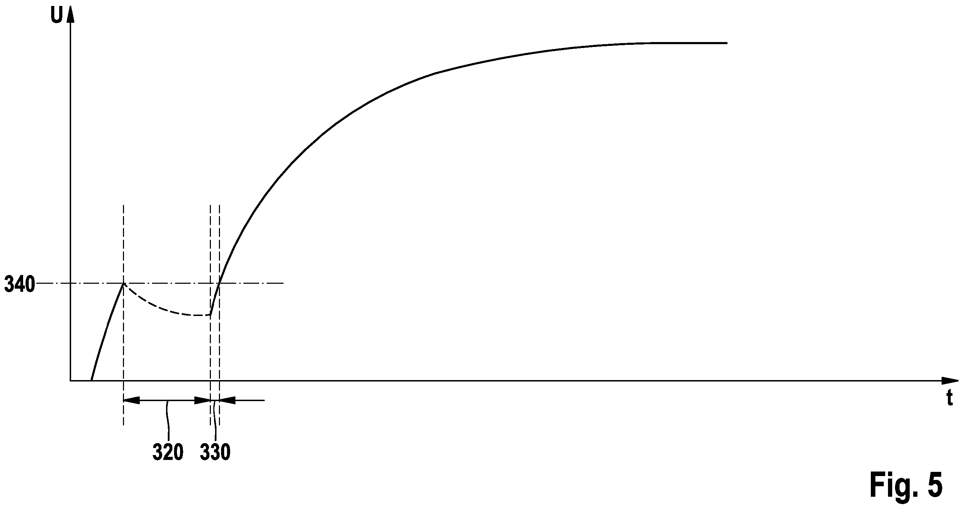

Überschreitet die zweite Zeitspanne 330 eine vorgegebene Zeitdauer, wie in Fig. 4 dargestellt, so ist dies ein Hinweis auf eine zu starke Selbstentladung des Kondensators 120. Ist dies der Fall, passt die Steuereinheit 170 das Ladeprogramm durch Herabsetzen des Wertes der ersten definierten Kondensatorspannung 310 auf eine geminderte Kondensatorspannung 340. Dieser Fall ist in Fig. 5 schematisch dargestellt.

Durch das Laden des Kondensators 120 gemäß den Fig.n 3 bis 5 erfolgt eine besonders schonende Reformierung des Kondensators 120. Das Anpassen des Ladeprogramms erfolgt bevorzugt im Rahmen des in Fig. 2 dargestellten Gesamtsystems.

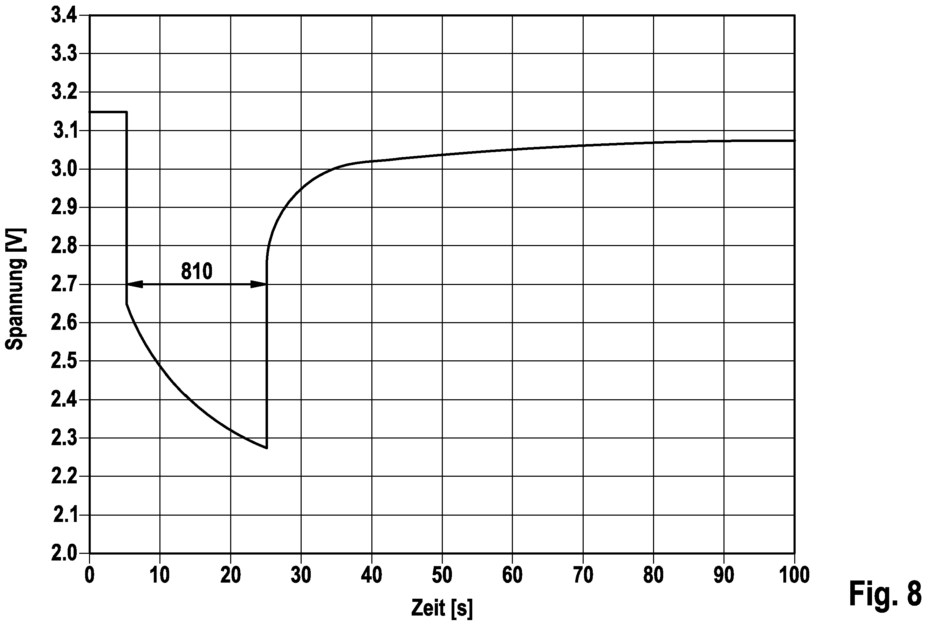

Ein Laden des Kondensators 120 gemäß den Fig. 3 bis 5 ist auch dann vorteilhaft, wenn der Kondensator 120 einen erhöhten Leckstrom aufweist. Zum Detektieren des erhöhten Leckstroms erfasst die Überwachungsvorrichtung 160 die Spannung der Batterie 110 während des Ladens des Kondensators 120. Zeigt die Batteriespannung dabei einen Verlauf wie in Fig. 8 dargestellt, so ist eine auffallend lange Ladezeit 810 indikativ für einen Kondensator 120 mit erhöhtem Leckstrom.

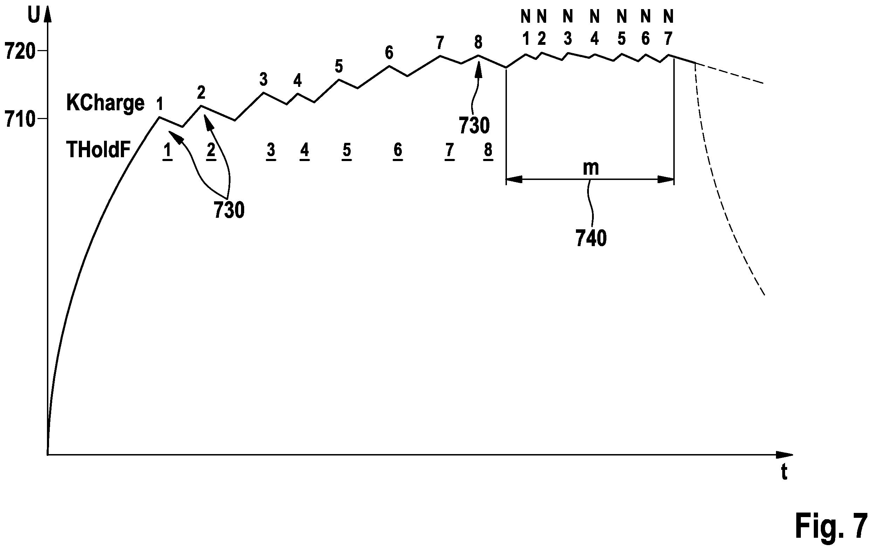

Alternativ zu den mit Fig. 3 bis Fig. 5 dargestellten Verläufen kann ein Kondensator mit erhöhtem Leckstrom auch gemäß einem Ladeverlauf nach Fig. 7 reformiert werden. Dabei lädt die Ladevorrichtung 130 den Kondensator 120 zunächst kontinuierlich auf einen Spannungspegel 710 deutlich unterhalb der Kondensatornennspannung. Danach lädt die Ladevorrichtung 130 den Kondensator 120 stufenweise (730), bis die KondensatorNennspannung 720 erreicht ist. Die Ladevorrichtung 130 hält die KondensatorNennspannung 720 durch zyklisches Nachladen 740 aufrecht. Die Parameter für eine Reformierung nach Fig. 7 sind stark abhängig vom jeweiligen Fehler des Kondensators 120 und werden bevorzugt von der Auswerteeinheit 190 festgesetzt.

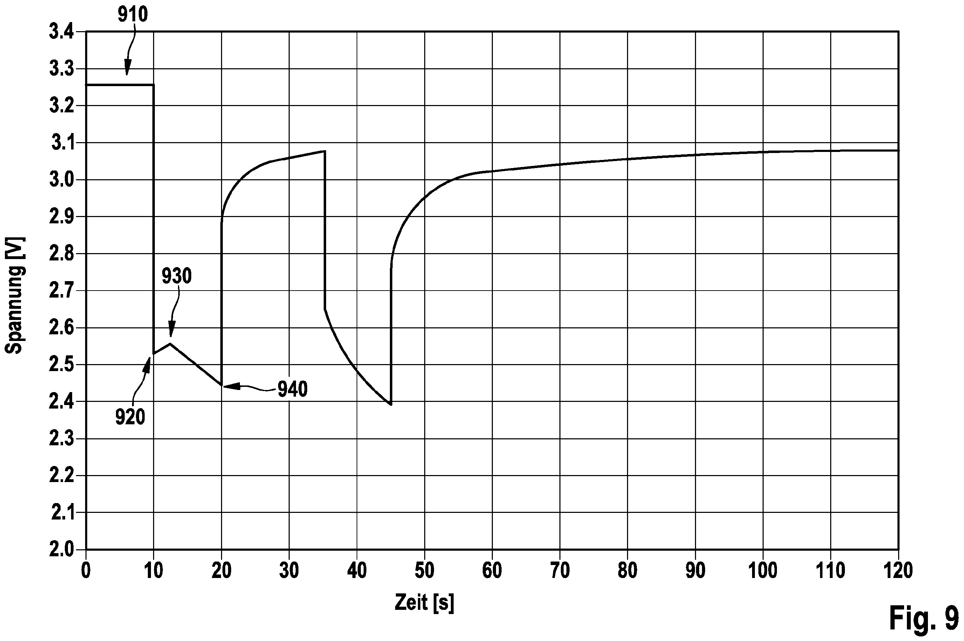

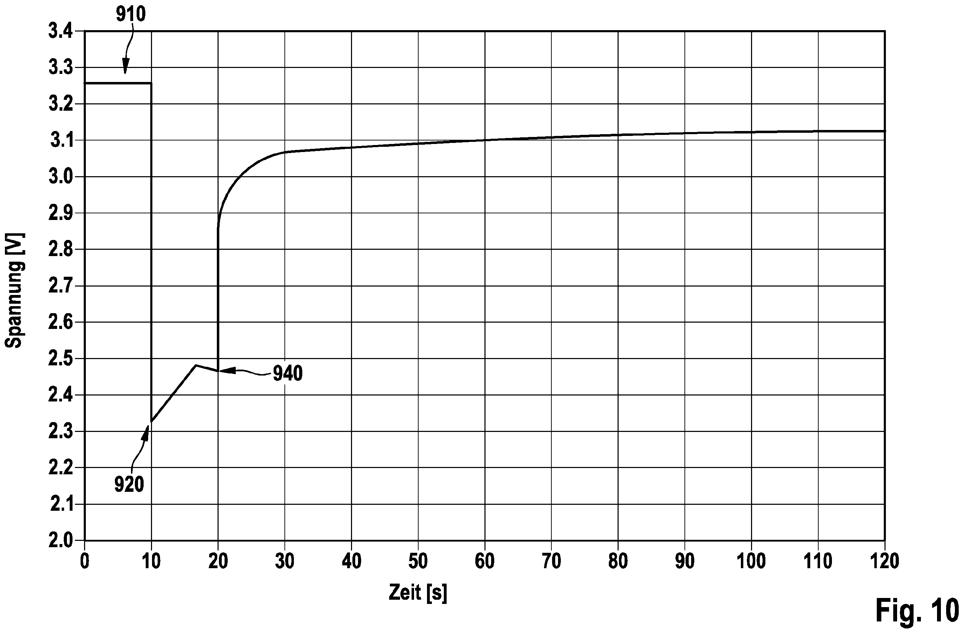

In den Figuren 9 und 10 sind zeitliche Verläufe der Spannung der Batterie 110 während eines Ladens des Kondensators 120 dargestellt. Die Überwachungsvorrichtung 160 ist ausgebildet, derartige Spannungsverläufe zu erfassen. In Fig. 9 ist der Batteriespannungsverlauf bei zwei aufeinanderfolgenden Ladevorgängen dargestellt. Eine Ruhespannung 910 der Batterie 110 bricht mit Beginn des Ladevorgangs, bedingt durch einen Spannungsabfall an einem Innenwiderstand der Batterie 110 und durch elektrochemische Prozesse, auf einen Anfangswert 920 ein. Während des ersten Ladevorgangs kommt es zunächst aufgrund chemischer Prozesse innerhalb der Batterie 110 zu einem leichten Spannungsanstieg 930. Dieser Spannungsanstieg 930 ist Ausdruck einer leichten Spannungsverzögerung. Eine Endspannung 940 liegt in diesem Fall deutlich unterhalb des Anfangswerts 920.

Beim zweiten Ladevorgang zeigt die Batterie 110 einen normalen Spannungsverlauf mit stetiger Abnahme der Batteriespannung während der Batteriebelastung, also während des Ladens des Kondensators 120. Um einer leichten Spannungsverzögerung entgegenzuwirken, passt die Steuereinheit 170 das Ladeprogramm durch Verkürzen der Reformierintervalle an.

In Fig. 10 ist ein von der Überwachungsvorrichtung 160 erfasster Verlauf der Spannung der Batterie 110 dargestellt, bei dem die Batterie 110 eine ausgeprägte Spannungsverzögerung aufweist. Ausgehend von der gewöhnlichen Ruhespannung 910 der Batterie bricht die Spannung mit Beginn des Ladevorgangs hier auf einen untypisch niedrigen Anfangswert 920 ein und steigt dann während des Ladens des Kondensators 120 an. In diesem Fall liegt der Endwert 940 der Batteriespannung deutlich oberhalb des Anfangswertes 920. Ein in Fig. 10 dargestellter Spannungsverlauf ist ein eindeutiger Indikator für eine ausgeprägte Spannungsverzögerung und damit ein Hinweis auf einen stark erhöhten Reformierbedarf.

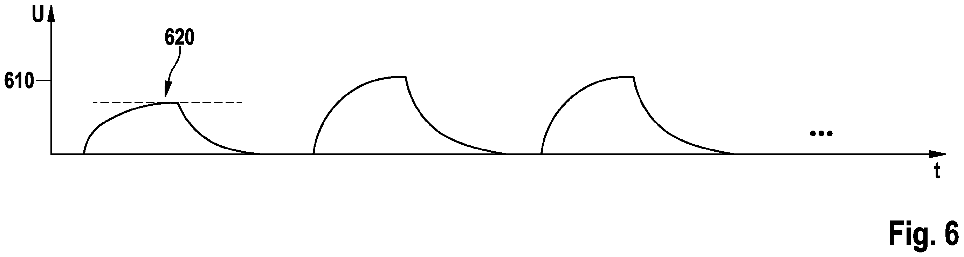

Die Auswerteeinheit 190 verursacht auf das Detektieren einer ausgeprägten Spannungsverzögerung hin ein Anpassen des Ladeprogramms, so dass sich ein in Fig. 6 dargestellter Verlauf der Kondensatorspannung ergibt. Demnach passt die Steuereinheit 170 das Ladeprogramm derart an, dass die Ladevorrichtung 130 den Kondensator 120 durch eine Vielzahl aufeinanderfolgender Ladezyklen mit einer verminderten Ladespannung lädt und dabei einen der Ladezyklen abbricht, falls der Wert der Kondensatorspannung in einer vorbestimmten Zeitspanne nicht eine vorgegebenen Schwellenwert 610 der Kondensatorspannung erreicht. Letzteres ist im Ladezyklus 620 der Fall.

Dieses Ladeprogramm bietet den Vorteil, dass mit einer moderaten Batteriebelastung eine Oxidschicht, die zum Ausbilden der Spannungsverzögerung führt, systematisch reformiert werden kann.

### Bezugszeichenliste

- 100: Implantat
- 110: Batterie
- 120: Kondensator
- 130: Ladevorrichtung
- 140: Schaltanordnung
- 150: Schockelektroden
- 160: Überwachungsvorrichtung
- 170: Steuereinheit
- 180: Senderempfänger
- 185: Schnittstelle des Senderempfängers
- 190: Auswerteeinheit
- 195: Schnittstelle der Auswerteeinheit
- 200: Datennetz
- 210: Fernüberwachungsserver
- 220: Person
- 230: Datenbank
- 240: Programmiergerät
- 250: Implantat ohne Anschluss an ein Datennetz
- 310: erste definierte Kondensatorspannung
- 320: erste definierte Zeitspanne
- 330: zweite Zeitspanne
- 610: ein vorgegebener Schwellenwert der Kondensatorspannung
- 620: Ladezyklus
- 710: Kondensatorspannung deutlich unterhalb der Kondensatornennspannung
- 720: Kondensatornennspannung
- 730: stufenweises Laden des Kondensators
- 740: zyklisches Nachladen des Kondensators
- 910: Ruhespannung
- 920: Anfangswert der Batteriespannung beim Laden des Kondensators
- 930: Spannungsanstieg beim Laden des Kondensators
- 940: Endwert der Batteriespannung nach Abschluss des Ladens des Kondensators
- 810: überhöhte Ladezeit

## Patentansprüche

1. Wartungssystem zum Warten einer Energiespeicheranordnung (110, 120, 130) eines Implantats (100), wobei
- die Energiespeicheranordnung (110, 120, 130) eine Batterie (110), einen Kondensator (120) und eine Ladevorrichtung (130) aufweist, die ausgebildet ist, dem Kondensator (120) gemäß einem Ladeprogramm elektrische Ladung von der Batterie (110) zuzuführen und
das Wartungssystem umfasst
- eine im Implantat (100) enthaltene Überwachungsvorrichtung (160), die ausgebildet ist, einen Wert eines physikalischen Parameters der Energiespeicheranordnung (110, 120, 130) zu erfassen und bereitzustellen;
- eine Auswerteeinheit (190) zum Bewerten des erfassten Wertes des physikalischen Parameters, die ausgebildet ist, in Abhängigkeit des erfassten Wertes des physikalischen Parameters ein Bewertungsergebnissignal zu erzeugen; **gekennzeichnet durch**
- einen im Implantat (100) enthaltenen Senderempfänger (180), der ausgebildet ist, den erfassten Wert des physikalischen Parameters an die Auswerteeinheit (190) zu versenden und das von der Auswerteeinheit (190) in Abhängigkeit des erfassten Wertes des physikalischen Parameters erzeugte Bewertungsergebnissignal zu empfangen und bereitzustellen; und
- eine im Implantat (100) enthaltene Steuereinheit (170) zum Steuern der Ladevorrichtung (130) und der Überwachungsvorrichtung (160), die ausgebildet ist, das Ladeprogramm in Abhängigkeit des Bewertungsergebnissignals anzupassen.

2. Wartungssystem nach Anspruch 1, bei dem die Auswerteeinheit (190) ausgebildet ist, eine Vielzahl von Werten des physikalischen Parameters von Energiespeicheranordnungen einer Vielzahl verschiedener Implantate zu empfangen und das Bewertungsergebnissignal im Rahmen einer Gesamtbewertung der Vielzahl Werte zu erzeugen.

3. Wartungssystem nach einem der vorstehenden Ansprüche, bei dem die Auswerteeinheit (190) über ein Patientengerät und dem Senderempfänger (180) mit der Überwachungsvorrichtung (160) und der Steuereinheit (170) wirkverbunden ist.

4. Wartungssystem nach einem der vorstehenden Ansprüche, bei dem die Steuereinheit (170) ausgebildet ist, das Ladeprogramm in Abhängigkeit des Bewertungsergebnissignals durch Änderung einer Ladespannung und/oder eines Ladeintervalls und/oder eines Ladestroms anzupassen.

5. Wartungssystem nach einem der vorstehenden Ansprüche, bei dem die Auswerteeinheit (190) ausgebildet ist, zum Erzeugen des Bewertungsergebnissignals auf eine Datenbank (230) mit Herstellungsdaten zuzugreifen, welche den betreffenden Implantatstyp charakterisieren.

6. Wartungssystem nach einem der vorstehenden Ansprüche, bei dem die Auswerteeinheit (190) im Implantat (100) enthalten ist.

7. Wartungssystem nach einem der vorstehenden Ansprüche, bei dem die Steuereinheit (170) ausgebildet ist, die Ladevorrichtung (130) und die Überwachungsvorrichtung (160) derart zu steuern, dass ein periodisches Laden des Kondensators (120) nach dem folgenden Ladeprogramm erfolgt:
- Initiieren des Ladens des Kondensators (120) durch die Ladevorrichtung (130);
- Erfassen einer elektrischen Spannung des Kondensators (120) mittels der Überwachungsvorrichtung (160);
- Unterbrechen des Ladevorgangs für eine erste definierte Zeitspanne (320) nach einem Erreichen einer ersten definierten Kondensatorspannung (310) des Kondensators (120);
- Fortsetzen des Ladens des Kondensators (120) nach Ablauf der ersten Zeitspanne (320);
- Erfassen des Wertes des physikalischen Parameters in Gestalt einer zweiten Zeitspanne (330), welche vom Fortsetzungsbeginn bis zu einem erneuten Erreichen der ersten definierten Kondensatorspannung (310) andauert, mittels der Überwachungsvorrichtung (160);
- Versenden des Wertes der erfassten zweiten Zeitspanne an die Auswerteeinheit (190) mittels des Senderempfängers (180);
- Bewerten des Wertes der zweiten Zeitspanne (330) und Bereitstellen des Bewertungsergebnissignals durch die Auswerteeinheit (190);
- Empfangen des Bewertungsergebnissignals mittels des Senderempfängers (180) und Anpassen des Ladeprogramms in Abhängigkeit des Bewertungsergebnissignals mittels der Steuereinheit (170).

8. Wartungssystem nach Anspruch 7, bei dem die Steuereinheit (170) ausgebildet ist, auf den Empfang des Bewertungsergebnissignals hin das Ladeprogramm durch Mindern der ersten definierten Kondensatorspannung anzupassen, falls das Bewertungsergebnissignal anzeigt, dass die zweite Zeitspanne (330) eine vorgegebene Zeitdauer übersteigt.

9. Wartungssystem nach Anspruch 8, bei dem die Steuereinheit (170) ausgebildet ist, nach dem Mindern der ersten definierten Kondensatorspannung und einem erneuten Überschreiten der vorgegebenen Zeitdauer die geminderte erste definierte Kondensatorspannung (340) erneut zu mindern, bis die zweite Zeitdauer die vorgegebene Zeitdauer nicht mehr überschreitet.

10. Wartungssystem nach einem der vorstehenden Ansprüche, bei dem
- die Überwachungsvorrichtung (160) ausgebildet ist, einen Verlauf eines Wertes einer an der Batterie (110) anliegenden Batteriespannung als physikalischen Parameter während eines Ladevorgangs des Kondensators (120) zu erfassen und
- die Steuereinheit (170) ausgebildet ist, das Ladeprogramm bei einem Bewertungsergebnissignal, das aufgrund des erfassten Batteriespannungsverlaufs indikativ für einen Wartungsbedarf der Batterie (110) ist, anzupassen.

11. Wartungssystem nach Anspruch 10, bei dem die Steuereinheit (170) ausgebildet ist, das Ladeprogramm derart anzupassen, dass die Ladevorrichtung (130) den Kondensator (110) durch eine Vielzahl aufeinanderfolgender Ladezyklen mit einer verminderten Ladespannung lädt und dabei einen der Ladezyklen abbricht, falls der Wert der Kondensatorspannung in einer vorbestimmten Zeitspanne nicht einen vorbestimmten Schwellenwert (610) erreicht.

12. Wartungssystem nach einem der vorstehenden Ansprüche, bei dem die Steuereinheit (170) ausgebildet ist, das Ladeprogramm auf einen Empfang des Bewertungsergebnissignals hin derart anzupassen, dass die Ladevorrichtung (130) den Kondensator (120) auf einen Spannungspegel deutlich unterhalb einer Kondensatornennspannung kontinuierlich lädt, sodann stufenweise lädt (730), bis die Kondensatornennspannung erreicht ist und schließlich die Kondensatornennspannung durch zyklisches Nachladen (740) aufrechterhält, falls das Bewertungsergebnissignal indikativ für einen überhöhten Leckstrom des Kondensators (120) ist.

## Claims

1. A maintenance system for maintaining an energy accumulator arrangement (110, 120, 130) of an implant (100),
- the energy accumulator arrangement (110, 120, 130) comprising a battery (110), a capacitor (120), and a charging device (130) designed to supply an electric charge from the battery (110) to the capacitor (120) in accordance with a charging program, and
the maintenance system comprising
- a monitoring device (160), present in the implant (100), which is designed to capture and provide a value of a physical parameter of the energy accumulator arrangement (110, 120, 130), and
- an evaluation unit (190) for evaluating the captured value of the physical parameter, which is designed to generate an evaluation result signal as a function of the captured value of the physical parameter,
**characterized by**
- a transceiver (180), present in the implant (100), which is designed to send the captured value of the physical parameter to the evaluation unit (190), and to receive and provide the evaluation result signal generated by the evaluation unit (190) as a function of the captured value of the physical parameter; and
- a control unit (170), present in the implant (100), for controlling the charging device (130) and the monitoring device (160), the control unit being designed to adapt the charging program as a function of the evaluation result signal.

2. The maintenance system according to claim 1, wherein the evaluation unit (190) is designed to receive a plurality of values of the physical parameter of energy accumulator arrangements of a plurality of different implants and to generate the evaluation result signal as part of an overall evaluation of the plurality of values.

3. The maintenance system according to any one of the preceding claims, wherein the evaluation unit (190) is operatively connected to the monitoring device (160) and the control unit (170) by way of a patient device and the transceiver (180).

4. The maintenance system according to any one of the preceding claims, wherein the control unit (170) is designed to adapt the charging program as a function of the evaluation result signal by changing a charging voltage and/or a charging interval and/or a charging current.

5. The maintenance system according to any one of the preceding claims, wherein the evaluation unit (190) is designed to access a database (230) comprising manufacturing data characterizing the particular implant type so as to generate the evaluation result signal.

6. The maintenance system according to any one of the preceding claims, wherein the evaluation unit (190) is present in the implant (100).

7. The maintenance system according to any one of the preceding claims, wherein the control unit (170) is designed to control the charging device (130) and the monitoring device (160) in such a way that periodic charging of the capacitor (120) takes place in accordance with the following charging program:
- initiating the charging of the capacitor (120) by the charging apparatus (130);
- capturing an electric voltage of the capacitor (120) by means of the monitoring device (160);
- interrupting the charging process for a first defined time period (320) after a first defined capacitor voltage (310) of the capacitor (120) has been reached;
- continuing the charging of the capacitor (120) after expiration of the first time period (320);
- capturing the value of the physical parameter in the form of a second time period (330), which lasts from the start of continuation until the first defined capacitor voltage (310) is reached again, by means of the monitoring device (160);
- sending the value of the captured second time period to the evaluation unit (190) by means of the transceiver (180);
- evaluating the value of the second time period (330) and providing the evaluation result signal by the evaluation unit (190); and
- receiving the evaluation result signal by means of the transceiver (180) and adapting the charging program as a function of the evaluation result signal by means of the control unit (170).

8. The maintenance system according to claim 7, wherein the control unit (170) is designed, upon receiving the evaluation result signal, to adapt the charging program by reducing the first defined capacitor voltage if the evaluation result signal indicates that the second time period (330) exceeds a predefined time period.

9. The maintenance system according to claim 8, wherein the control unit (170), after lowering the first defined capacitor voltage and again exceeding the predefined time period, is designed to again lower the lowered first defined capacitor voltage (340) until the second time period no longer exceeds the predefined time period.

10. A maintenance system according to any one of the preceding claims, wherein
- the monitoring device (160) is designed to capture a curve of a value of battery voltage present at the battery (110) as a physical parameter during a charging process of the capacitor (120), and
- the control unit (170) is designed to adapt the charging program in the case of an evaluation result signal which is indicative of a need for maintenance of the battery (110) based on the captured battery voltage curve.

11. The maintenance system according to claim 10, wherein the control unit (170) is designed to adapt the charging program in such a way that the charging device (130) charges the capacitor (110) by way of a plurality of consecutive charging cycles using a reduced charging voltage, and aborts one of the charging cycles if the value of the capacitor voltage does not reach a predetermined threshold value (610) in a predetermined time period.

12. The maintenance system according to any one of the preceding claims, wherein the control unit (170) is designed to adapt the charging program, upon receiving the evaluation result signal, in such a way that the charging device (130) continuously charges the capacitor (120) to a voltage level which is significantly below a rated capacitor voltage, then charges it incrementally (730) until the rated capacitor voltage has been reached, and finally maintains the rated capacitor voltage by way of cyclical recharging (740) if the evaluation result signal is indicative of increased leakage current of the capacitor (120).

## Revendications

1. Système d'entretien pour l'entretien d'un agencement de stockage d'énergie (110, 120, 130) d'un implant (100), dans lequel
- l'agencement de stockage d'énergie (110, 120, 130) présente une pile (110), un condensateur (120) et un dispositif de charge (130), qui est conçu pour amener au condensateur (120) la charge électrique selon un programme de charge à partir de la pile (110), et
le système d'entretien comprend
- un dispositif de surveillance (160) contenu dans l'implant (100) qui est conçu pour détecter et mettre à disposition une valeur d'un paramètre physique de l'agencement de stockage d'énergie (110, 120, 130) ;
- une unité d'évaluation (190) pour l'évaluation de la valeur détectée du paramètre physique, qui est conçue pour générer un signal d'évaluation de résultat en fonction de la valeur détectée du paramètre physique ;
**caractérisé par**
- un émetteur-récepteur (180) contenu dans l'implant (100), qui est conçu pour transférer la valeur détectée du paramètre physique vers l'unité d'évaluation (190) et pour recevoir et mettre à disposition le signal d'évaluation de résultat généré par l'unité d'évaluation (190) en fonction de la valeur détectée du paramètre physique ; et
- une unité de commande (170) contenue dans l'implant (100) pour la commande du dispositif de charge (130) et du dispositif de surveillance (160), qui est conçue pour adapter le programme de charge en fonction du signal d'évaluation de résultat.

2. Système d'entretien selon la revendication 1, chez lequel l'unité d'évaluation (190) est conçue pour recevoir un grand nombre de valeurs de paramètres physiques d'agencement de stockage d'énergie d'un grand nombre d'implants divers et pour générer le signal d'évaluation de résultat dans le cadre d'une évaluation complète du grand nombre de valeurs.

3. Système d'entretien selon l'une des revendications précédentes, chez lequel l'unité d'évaluation (190) est reliée de manière opérationnelle par le biais d'un appareil d'un patient et de l'émetteur-récepteur (180) avec le dispositif de surveillance (160) et l'unité de commande (170).

4. Système d'entretien selon l'une des revendications précédentes, chez lequel l'unité de commande (170) est conçue pour adapter le programme de charge en fonction du signal d'évaluation de résultat par une modification de la tension de charge et/ou de l'intervalle entre les charges, et/ou d'un courant de charge.

5. Système d'entretien selon l'une des revendications précédentes, chez lequel l'unité d'évaluation (190) est conçue pour avoir accès à une banque de données (230) avec des données de fabrication, lesquelles caractérisent le type d'implant concerné pour la génération du signal d'évaluation de résultat.

6. Système d'entretien selon l'une des revendications précédentes, chez lequel l'unité d'évaluation (190) est contenue dans l'implant (100).

7. Système d'entretien selon l'une des revendications précédentes, chez lequel l'unité de commande (170) est conçue pour commander le dispositif de charge (130) et le dispositif de surveillance (160) de telle manière qu'une charge périodique du condensateur (120) a lieu selon le programme de charge suivant :
- initialisation de la charge du condensateur (120) par le dispositif de charge (130) ;
- détection d'une tension électrique du condensateur (120) au moyen du dispositif de surveillance (160) ;
- interruption du processus de charge pour un premier laps de temps (320) défini après avoir atteint une première tension de condensateur (310) définie du condensateur (10) ;
- poursuite de la charge du condensateur (120) après le déroulement du premier laps de temps (320) ;
- détection de la valeur du paramètre physique sous la forme d'un deuxième laps de temps (330), lequel dure depuis le début de la poursuite jusqu'à l'atteinte de la première tension de condensateur (310) définie, au moyen du dispositif de surveillance (160) ;
- envoi de la valeur du deuxième laps de temps détectée vers l'unité d'évaluation (190) au moyen de l'émetteur-récepteur (180) ;
- évaluation de la valeur du deuxième laps de temps (330) et fourniture du signal d'évaluation de résultat par l'unité d'évaluation (190) ;
- réception du signal d'évaluation de résultat au moyen de l'émetteur-récepteur (180) et adaptation du programme de charge en fonction du signal d'évaluation de résultat au moyen de l'unité de commande (170).

8. Système d'entretien selon la revendication 7, chez lequel l'unité de commande (170) est conçue pour adapter le programme de charge suite à la réception du signal d'évaluation de résultat par une diminution de la première tension de condensateur définie dans le cas où le signal d'évaluation de résultat indique que le deuxième laps de temps (330) dépasse une durée prédéfinie.

9. Système d'entretien selon la revendication 8, chez lequel l'unité de commande (170) est conçue, après la diminution de la première tension de condensateur définie et un nouveau dépassement de la durée prédéfinie, pour diminuer de nouveau la première tension de condensateur (340) définie diminuée jusqu'à ce que la deuxième durée ne dépasse plus la durée prédéfinie.

10. Système d'entretien selon l'une des revendications précédentes, chez lequel
- le dispositif de surveillance (160) est conçu pour détecter une évolution d'une valeur d'une tension de pile appliquée sur la pile (110) en tant que paramètre physique pendant un processus de charge du condensateur (120) et
- l'unité de commande (170) est conçue pour adapter le programme de charge lors d'un signal d'évaluation de résultat qui est indicatif d'un besoin d'entretien de la pile (110) en raison de l'évolution de la tension de pile détectée.

11. Système d'entretien selon la revendication 10, chez lequel l'unité de commande (170) est conçue pour adapter le programme de charge de telle manière que le dispositif de charge (130) charge le condensateur (110) par un grand nombre de cycles de charge se succédant avec une tension de charge réduite et ce faisant, interrompt l'un des cycles de charge dans le cas où la valeur de la tension de condensateur n'atteint pas une valeur de seuil (610) prédéterminée dans un laps de temps prédéterminé.

12. Système d'entretien selon l'une des revendications précédentes, chez lequel l'unité de commande (170) est conçue pour adapter le programme de charge suite à une réception du signal d'évaluation de résultat de telle manière que le dispositif de charge (130) charge continuellement le condensateur (120) à un niveau de tension nettement en dessous d'une tension nominale de condensateur, charge ainsi par étapes jusqu'à ce que la tension de condensateur soit atteinte et que finalement la tension de condensateur se maintienne par la recharge (740) cyclique dans le cas où le signal d'évaluation de résultat est indicateur d'un courant de fuite surdimensionné du condensateur (120).
